# EUROPEAN PATENT SPECIFICATION

(11) **EP 4 378 490 B1**
(45) Date of publication and mention of the grant of the patent: **04.02.2026**
(21) Application number: 22383162.9
(22) Date of filing: 01.12.2022
(51) Int. Cl.: A61L 24/00, A61L 24/02, A61L 24/06

(54) **HAEMOSTATIC COMPOSITION**
HÄMOSTATISCHE ZUSAMMENSETZUNG
COMPOSITION HÉMOSTATIQUE

(43) Date of publication of application: 05.06.2024
(73) Proprietor: Ascil Proyectos SL, 08860 Barcelona (ES)
(72) Inventor: Cherif-Cheikh, Roland, 08860 Barcelona (ES); Cardús Malaspina, Merce, 08758 Barcelona (ES); Pérez López, Montserrat, 08037 Barcelona (ES); Alegre Fernandez, Marta, 08221 Barcelona (ES)
(74) Representative: Cabinet Grosset-Fournier & Demachy

(56) References cited:
- WO-A1-2013/004683
- CN-A- 107 720 854
- CN-B- 107 261 193
- US-A- 4 260 597
- US-A- 4 395 398

## Description

### Technical field

The present invention relates to a haemostatic composition which comprises aluminium and ferric salts as active agents, and which provides improved haemostatic effect.

### State of the art

The use of topical haemostatic agents is common practice after minor surgical procedures, for example, in dermatology, odontology or gynaecology, in order to stop bleeding and also to stimulate the wound healing process.

A classic well-known example of topical haemostatic agent is the so-called Monsel's solution, which is a 20% ferric subsulphate aqueous solution first disclosed by Leo Monsel in 1856 and which is typically prepared by reacting ferrous sulphate with sulfuric acid and nitric acid. Haemostasis is thought to result from denaturation and agglutination of proteins (such as fibrinogen) by ferric ions, boosted by the low pH and the subsulphate group (Larson P.O., Topical haemostatic agents for dermatologic surgery, J. Dermatol. Surg. Oncol., 1988, 14(6), 623-632).

There are variants of Monsel's solution, in the form of paste or gel. Monsel's paste, for example, contains ferric sulphate base, ferrous sulphate powder, sterile water and glycerol starch, while Monsel's gel is prepared from ferrous sulphate, sulfuric acid, nitric acid, hydroxypropyl guar gum and water (Solución de Monsel, Farmacotecnia, boletin informativo SEFH, 2011, 1(1), 4-5).

Another ferric salt, namely, ferric chloride, has also been shown to provide effective haemostasis for controlling superficial bleeding, in particular, used in the form of aqueous solution having a concentration of 5 to 50% in active principle. Such compositions are disclosed, for example, in the article Nouri et al., The effect of ferric chloride on superficial bleeding, Trauma Mon., 2015, 20 (1), e18042.

Another haemostatic agent namely aluminium chloride was used as a solution in water or other solvents, such as ethanol, ether or glycerol. The haemostatic effect in this case may be related to its hydrolysis to HCl. Other metal ions have been used as well as haemostatic agents, such as silver nitrate or zinc chloride, for example (Larson P.O., *op. cit.*)*.*

There are several disclosures in the prior art about further variants of haemostatic compositions; in particular, some of those disclosures relate to the formulation of viscous compositions, in order to facilitate the handling and application of the composition and also to better affix it to the skin or mucosal area in need of treatment, thus providing improved haemostatic effect. The formulation of those viscous compositions, however, is not trivial, as the additional components must be compatible with the active metal salts and must also be stable in the typically highly acidic environment of such compositions.

In the international patent application WO2014/043743 viscous haemostatic compositions are disclosed comprising an ionic precipitating agent (which is a Monsel's solution, i.e. comprising ferric subsulphate), a mixture of a fatty alcohol (which is cetostearyl alcohol), an alcohol phosphate diester (which is dicetyl phosphate) and a monoester phosphate of an alkoxylated fatty alcohol (which is ceteth-10 phosphate) as gelling agent, and water as solvent.

The patent application US2009/0041858 discloses haemostatic compositions with greater viscosity comprising a haemostatic agent, which is, for example, ferric sulphate, and a nitrogen-containing polymer as gelling agent, namely, poly-2-ethyl-2-oxazoline or *N*-vinyl-2-pyrrolidone.

The international patent application WO96/25915 discloses a haemostatic composition which is particularly suitable for dental use, to stop bleeding during dental restorative and reconstructive procedures. The compositions disclosed comprise a haemostatic agent (ferric sulphate or aluminium chloride), deionized water as solvent, and an additional agent, which is preferably fumed silica, polyethylene glycol, or mixtures thereof, for reducing the aggressiveness of the haemostatic agent on dental tissue. Furthermore, the silica component causes the haemostatic composition to become more viscous for improved application control.

The US patent 5,575,995 discloses a haemostatic gel comprising ferric subsulphate, water and glycerin, which is a thickening agent and a solubilizer for the subsulphate. The compositions may also contain polyvinyl pyrrolidone (PVP) as film former or gelling agent.

The US patent 5,011,693 discloses haemostatic compositions comprising either ferric sulphate or aluminium chloride and colloidal silicon dioxide as gelling agent.

Some prior art references disclose formulations where different haemostatic agents are combined. Thus, for example, the US patent 6,652,840 discloses a composition in the form of paste which is particularly suitable for bleeding control in dental procedures which comprises aluminium chloride, ferric sulphate, regenerated oxidized cellulose, aluminium ammonium sulphate, absorbable gelatin and a solvent.

International application WO2003/063922 relates to haemostatic agents comprising at least one porous polyvinyl alcohol (PVA) in solid form, selected from the group consisting of uncross-linked PVA with a molecular weight of between 15,000 and 400,000, cross-linked PVA and mixtures thereof.

On the other hand, the international patent application WO2013/004683 discloses a haemostatic agent which is particularly intended for use in surgical procedures which comprises 20% of aluminium chloride hexahydrate, 6% or ferric chloride, 0.9% of methylcellulose as viscosifying agent and 73.1% of water as solvent. The composition exhibited coagulant, cauterizing and dehydrating activity in cut hepatic parenchyma.

Despite the different proposals disclosed so far in the prior art, there is still the need of providing further haemostatic compositions which are more effective, are suitable to stop bleeding in different tissues and for different procedures, have optimal viscosity for an easier handling and application and have improved long-term stability.

In sum, at the priority date of the application, it would appear from the prior art that combining several active principles like metal chlorides and augmenting the viscosity of the haemostatic compositions with materials like polyvinyl alcohol (PVA) was a promising route to explore to arrive at improved haemostatic compositions. However, the prior art was deterring the one skilled in the art to add PVA to combinations of metal chlorides because on the one hand said metal compositions were known to be highly acidic and on the other hand, PVA was widely known to be unstable under such acidic conditions (see Polyvinyl Alcohol monograph op. cit.).

In other words, there was a prejudice against substituting, in the prior art compositions of WO2013/004683, PVA whose use was taught in WO2003/063922, for methylcellulose as a viscosizing agent because said compositions of WO2013/004683 are strongly acidic and PVA was known to be unstable in such conditions.

By conceiving haemostatic compositions comprising ferric chloride aluminum chloride and polyvinyl alcohol the inventors overcame a prejudice and surprisingly arrived at stable and more efficacious haemostatic compositions.

### Object of the invention

The scope of this invention is defined by the claims. Embodiments in the description relating to methods of treatment are not covered by the claims. The object of the present invention is a haemostatic composition, as defined in the appended claims.

Another aspect of the invention is the composition for use in promoting haemostasis.

### Description of the Drawings

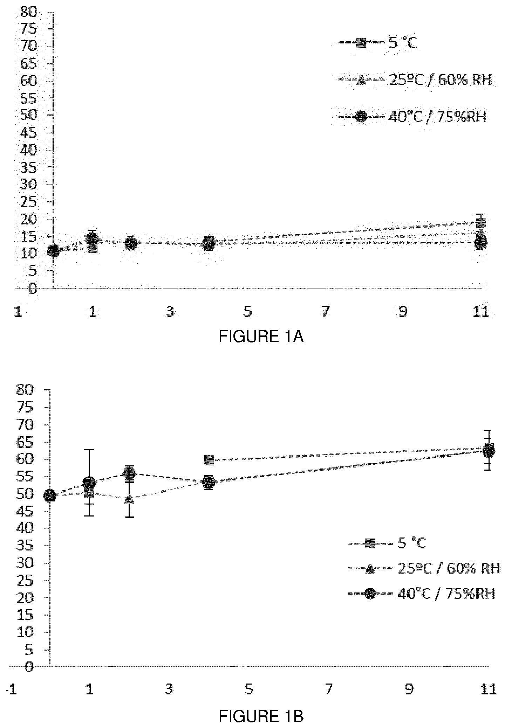

Figure 1 shows the injection force needed to deliver at a specific flow rate the composition of Example 1, measured at different time points of storage of the composition, using a syringe with different needles, as described in Example 2.

Line A corresponds to a sample stored at 25º C and 60% RH, line B corresponds to a sample stored at 40ºC and 75% RH and line C corresponds to a sample stored at 5ºC. Figure 1A shows the results of this assay using a 21 G needle, while Figure 1B shows the results of the assay using a 23G needle.

In the Figures, the y-axis represents the force (in newtons, N) and the x-axis represents the time of storage of the composition (in weeks).

### Detailed description of the invention

The object of the present invention is a haemostatic composition comprising:
a) ferric chloride or a hydrate thereof in an amount comprised in the range 10-25 wt%, expressed as equivalent amount of ferric chloride;
b) aluminium chloride or a hydrate thereof in an amount comprised in the range 5-20 wt%, expressed as equivalent amount of aluminium chloride;
c) polyvinyl alcohol in an amount comprised in the range 5-15 wt%

and the pharmaceutically acceptable excipients ethanol in an amount comprised in the range 10-25 wt% and water, wherein the composition has a pH comprised between 1 and 3, and
wherein the percentages are referred to the total weight of the composition.

The inventors of the present invention have developed a haemostatic composition in the form of a viscous solution, which comprises ferric chloride and aluminium chloride as haemostatic agents, water and ethanol as solvents and polyvinyl alcohol as viscosifying agent. Surprisingly, this specific combination gives rise to optimal properties to the composition, in terms of viscosity and handling and haemostatic efficacy; furthermore, it is remarkably stable despite being highly acidic and despite having high concentration of the haemostatic salts.

In the present description, as well as in the claims, the singular expressions, generally preceded by the articles "a", "an" or "the", are meant to include also the plural forms, unless the context clearly indicates otherwise.

The terms "about" or "approximately" referred to amounts, as used herein, are meant to include a certain deviation of the qualified amount, namely of ±5%. Numeric ranges defined by lower and upper endpoints are meant to include also said stated endpoints. Unless otherwise indicated, the percentages (%) are expressed as weight/weight percentages (also represented as wt%)

### Haemostatic active agents

The composition of the present invention comprises the combination of ferric chloride and aluminium chloride as haemostatic agents.

Ferric chloride, also known as iron (III) chloride, has the formula FeCl₃ It may be used as such for preparing the composition of the invention, i.e. in anhydrous form, or may be used as a hydrate thereof, for example, as the hexahydrate (FeCl₃·6H₂O).

In one embodiment, ferric chloride hexahydrate is used.

The percentage of ferric chloride in the composition is comprised in the range 10-25 wt%, preferably in the range 15-22 wt%, more preferably in the range 19-21 wt%, and still more preferably is about 20 wt%. Those percentages are referred to the total weight of the composition.

When a hydrate of ferric chloride is used, for example, the hexahydrate, the above percentages express the equivalent amount of the anhydrous form, which can be easily calculated based on the respective molecular weights.

Thus, for example, when the hexahydrate is used, a composition comprising 10-25 wt% of anhydrous ferric chloride means a composition comprising about 17-42 wt% of ferric chloride hexahydrate; or a composition comprising 20 wt% of anhydrous ferric chloride means a composition comprising about 33 wt% of ferric chloride hexahydrate.

Analogously, aluminium chloride (AlCl₃) may be used as such, i.e. in anhydrous form, or as a hydrate thereof, for example, as the hexahydrate (AlCl_{3̇}·6H₂O).

In one embodiment, aluminium chloride hexahydrate is used.

The percentage of aluminium chloride in the composition is comprised in the range 5-20 wt%, preferably comprised in the range 7-15 wt%, more preferably comprised in the range 8-12 wt%, and still more preferably is about 10 wt%. Those percentages are referred to the total weight of the composition.

When a hydrate of aluminium chloride is used, for example, the hexahydrate, the above percentages express the equivalent amount of the anhydrous form, which can be easily calculated based on the respective molecular weights.

Thus, for example, when the hexahydrate is used, a composition comprising 5-20 wt% of anhydrous aluminium chloride means a composition comprising about 9-36 wt% of aluminium chloride hexahydrate; or a composition comprising 10 wt% of anhydrous aluminium chloride means a composition comprising about 18 wt% of aluminium chloride hexahydrate.

Both ferric chloride and aluminium chloride, as well as hydrates thereof, are readily available from commercial sources.

### Polyvinyl alcohol

Polyvinyl alcohol (PVA) is another component of the composition of the invention, which is used as viscosifying agent.

The concentration of PVA in the composition is comprised in the range 5-15 wt%, preferably in the range 6-12 wt%, more preferably in the range 7-11 wt% and still more preferably in the range 8-10 wt%, wherein the percentages are relative to the total weight of the composition.

As is well-known in the art (see, for example, the Polyvinyl Alcohol monograph in: Handbook of Pharmaceutical Excipients, Sixth Edition, 2009, Rowe R.C., Sheskey P.J., Quinn M.E., ed.), PVA is available in different grades, which correspond to different molecular weights. Generally, it is available in molecular weights (MW) ranging from 20 000 to 200 000.

PVAs having a molecular weight within this range of MW are suitable to be used in the present composition.

In one embodiment, the PVA has a molecular weight comprised between 75 000 and 150 000, preferably comprised between 90 000 and 130 000, more preferably comprised between 110 000 and 120 000, and still more preferably of about 115 000.

As discussed in Example 2, the composition according to the invention comprising PVA as viscosifying agent remained stable in stability studies at 25ºC/60% RH and 40ºC/75% RH, for at least 3 weeks.

This was surprising because the present haemostatic composition is strongly acidic and has high concentration of inorganic salts, and the PVA is reported in the literature as being incompatible both with strong acids and with high concentration of inorganic salts (see Polyvinyl Alcohol monograph op. cit.).

### Solvent

The composition comprises ethanol and water as solvents.

Ethanol is used in a concentration comprised between 10 wt% and 25 wt%, and more preferably comprised between 15 wt% and 25 wt%, wherein the percentages are in weight, relative to the total weight of the composition.

The ethanol used to prepare the composition may contain a small proportion of water. In general, ethanol-water mixture comprising at least 95 % (v/v) of ethanol is used. Typically, for example, ethanol (96%) is used, which contains 95.1-96.9 % v/v of pure ethanol. Other types of ethanol of higher purity can also be used, for example, anhydrous ethanol, which contains not less than 99.5 % v/v of pure ethanol (see *Alcohol* monograph in the Handbook of Pharmaceutical Excipients, *op.cit.).*

The ethanol concentration ranges in the composition, as expressed above, are typically calculated on the basis of the total weight of the ethanol-water mixture used to prepare the composition, for example, based on the total weight of ethanol 96% (v/v) used in the composition.

Ethanol has the function of co-solvent and preservative in the composition.

The compositions of the invention contain ethanol as the solvent to be used with water.

Water is used as solvent of the composition. The amount of water used is adjusted up to the 100% of the composition according to the percentages of the other components as discussed above.

Water is typically purified water.

### Haemostatic composition

The haemostatic composition of the present invention is strongly acidic, with a pH value comprised between 1 and 3, preferably comprised between 1 and 2.

Optionally, the composition of the invention may comprise additional ingredients, for example, additional haemostatic active ingredients, additional cosolvents, preservatives and/or colouring agents, among others, for example.

Additional haemostatic active ingredients include, for example, other ferric salts, such as ferric sulphate or ferric subsulphate, among others; or other aluminium salts, such as aluminium sulphate, aluminium chlorohydrate, aluminium acetate or aluminium ammonium sulphate, for example. When additional ferric salts are included, preferably the amount is such that the total percentage of iron (III) ion in the composition is not higher than about 8.6 wt%. When additional aluminium salts are included, preferably the amount is such that the total percentage of aluminium ion in the composition is not higher than about 4 wt%.

In one embodiment, the composition essentially consists of aluminium chloride or a hydrate thereof, ferric chloride or a hydrate thereof, polyvinyl alcohol, ethanol and water, in the amounts discussed above; in this context, "essentially consists" means that the composition may still contain minor amounts (i.e. less than about 5 wt%, or preferably less than about 3 wt%, or still more preferably less than about 1 wt%, or still more preferably less than about 0.5 wt%) of other auxiliary excipients which do not interfere with the activity of the composition, for example, a preservative, such as benzalkonium chloride, a flavouring agent, and/or a colouring agent, for example.

In one embodiment, the composition consists of aluminium chloride or a hydrate thereof, ferric chloride or a hydrate thereof, polyvinyl alcohol, ethanol and water, in the amounts discussed above, i.e., it does not contain any additional ingredient.

In one embodiment, the composition of the invention comprises:
a) ferric chloride or a hydrate thereof in an amount comprised in the range 10-25 wt%, preferably comprised in the range 15-22 wt%, more preferably comprised in the range 19-21 wt% and still more preferably the amount is about 20 wt%, expressed as the equivalent amount of anhydrous ferric chloride;
b) aluminium chloride or a hydrate thereof in an amount comprised in the range 5-20 wt%; preferably comprised in the range 7-15 wt %, more preferably comprised in the range 8-12 wt%, and still more preferably the amount is about 10 wt%, expressed as the equivalent amount of anhydrous aluminium chloride;
c) polyvinyl alcohol in an amount comprised in the range 5-15 wt%, preferably comprised in the range 6-12 wt%, more preferably comprised in the range 7-11 wt% and still more preferably the amount comprised in the range 8-10 wt%;
d) ethanol, in an amount comprised in the range 10-25 wt% and still more preferably comprised in the range 15-25 wt%; and
e) water;
wherein the percentages are referred to the total weight of the composition.

In one particular embodiment, the composition essentially consists of ferric chloride or a hydrate thereof, aluminium chloride or a hydrate thereof, polyvinyl alcohol, ethanol and water. In another particular embodiment, the composition exactly consists of ferric chloride or a hydrate thereof, aluminium chloride or a hydrate thereof, polyvinyl alcohol, ethanol and water.

The composition may be prepared by simple mixture of the components, for example, by dissolving the ferric and aluminium salts in a mixture of water and ethanol, under stirring, and finally adding the polyvinyl alcohol.

### Uses

The haemostatic composition of the present invention is effective for the control of bleeding. It is a viscous fluid so it is easy to handle, it can be accurately applied over the area in need to be treated, and it remains conveniently affixed to the treated area thus boosting the haemostatic and healing effect. The composition is also stable, is well tolerated and does not produce irritation.

The haemostatic effect is achieved without any epithelial damage (without epithelial injury). It is an additional advantage of the product as opposed to, for example, electrocoagulation or coagulants such as silver nitrate, which produce sometimes significant necrosis.

Therefore, another aspect of the invention is the composition for use in promoting haemostasis, i.e., for stopping bleeding, at a site of blood loss in a patient in need thereof.

Another aspect described herein, but not part of the claimed invention, is a method of promoting haemostasis in a patient in need thereof comprising the step of topically administering the composition of the invention at the site of blood loss.

The composition is intended for topical use, i.e., to be applied and spread over an area of skin or mucosa in need of bleeding control. Types of mucosa suitable to be treated with the composition of the invention include, for example, oral, nasal, vaginal or anal mucosa.

The practical applications of the composition of the invention are wide and include every situation where rapid control of bleeding is required.

The bleeding is generally caused by a damage to the skin or mucosa and the underlying tissues, either by accidental injury or after medical or surgical procedures.

In particular, for example, the composition of the invention can be used for promoting haemostasis after minor surgical procedures, including biopsies and sutures; It is particularly suitable, for example, as haemostatic after biopsies, including skin biopsies, cervical biopsies or vulvar biopsies, for example.

Another common use is for stopping bleeding in different dental procedures. For example, it can be used in dental restorative and reconstructive procedures where bleeding control is required in order to take accurate impressions of the teeth.

Another practical application is for promoting haemostasis in superficial cuts and injuries, in front of wounds or skin erosions.

The product can be recommendable for patients treated with anticoagulants who have coagulation problems.

The composition of the invention may be available in any conventional mono- or multi-dose package, as are well-known in the art, including bottles, tubes or sachets, for example, among others.

Depending on the required site to be applied, it can be directly applied, optionally with the aid of a suitable device, such as a spatula, syringe or any other suitable applicator. It can be also be delivered impregnated in different supports, such as dressings, bandages, gauzes, or the like.

Furthermore, the product is easily administrable, and can be applied by the patient itself.

Next, some examples are provided with the purpose of illustrating the invention, but not limiting it.

### Examples

### Example 1.- Preparation of a haemostatic composition according to the invention

A haemostatic composition was prepared using the components disclosed in the following table:

| **Ingredient** | **Weight (g)** | **wt%** | **wt% (anhydrous salt)** |
|---|---|---|---|
| FeCl₃·6H₂O | 3.20 | 32.26 | 19.36 |
| AlCl₃·6H₂O | 1.80 | 18.15 | 10.02 |
| PVA | 1.00 | 10.08 | |
| Ethanol 96% | 1.95 | 19.66 | |
| Purified water | 1.97 | 19.86 | |
| **Total** | **9.92** | **100** | |

The composition was prepared according to the following procedure: FeCl₃·6H₂O was dissolved in ethanol 96% and AlCl₃·6H₂O was dissolved in H₂O. Both solutions were mixed in a bottle protected from light. PVA was then added to the solution and mixed for 5-10 min at 30-35ºC. Finally, agitation was left overnight at RT for complete dissolution.

The composition obtained was a viscous solution, of density 1.195g/L. The pH of the composition was of about 0-1.

### Example 2.- Stability assays

The stability of the composition prepared in Example 1 was assessed. In particular, an assay was performed to assess whether the viscosity of the composition remained stable over time.

For this purpose, three samples of the composition prepared in Example 1 were kept for 3 weeks, under different conditions:
- 25ºC and 60% RH (sample A)
- 40ºC and 75% RH (sample B)
- 5ºC (sample C)

The viscosity of the samples was measured periodically during this period. The changes in viscosity were assessed indirectly, by measuring the changes in the injection force (in N) required, using two needles of different diameter, namely 21G and 23G (for a constant flow rate of 20 mm/min).

The results of the comparative assays are graphically represented in Figure 1.

It was found that the viscosity of the solution remained stable in all the assayed conditions.

### Example 2.- Efficacy assays

The composition of Example 1 was used as haemostatic agent after cutaneous biopsies, and it was effective for avoiding bleeding and for boosting wound healing. Compared to a standard liquid Monsel's solution, the haemostatic performance was superior.

It was found particularly useful as haemostatic agent after punch biopsies, as the viscous solution remained inside the formed cavity, pressing the cavity walls and thus speeding the wound healing process.

## Claims

1. - Haemostatic composition **characterized in that** it comprises:
a) ferric chloride or a hydrate thereof in an amount comprised in the range 10-25 wt%, expressed as equivalent amount of ferric chloride;
b) aluminium chloride or a hydrate thereof in an amount comprised in the range 5-20 wt%, expressed as equivalent amount of aluminium chloride;
c) polyvinyl alcohol in an amount comprised in the range 5-15 wt% and the pharmaceutically acceptable excipients ethanol in an amount comprised in the range 10-25 wt% and water,
wherein the composition has a pH comprised between 1 and 3
wherein the percentages are referred to the total weight of the composition.

2. Composition according to claim 1, **characterized in that** the amount of ferric chloride or a hydrate thereof is comprised in the range 15-22 wt%.

3. - Composition according to claim 2, **characterized in that** the amount of ferric chloride or a hydrate thereof is comprised in the range 19-21 wt%.

4. - The composition according to any one of claims 1 to 3, **characterized in that** ferric chloride hexahydrate is used.

5. - The composition according to any one of claims 1 to 4, **characterized in that** the amount of aluminium chloride or a hydrate thereof is comprised in the range 7-15 wt%.

6. - The composition according to claim 5, **characterized in that** the amount of aluminium chloride or a hydrate thereof is comprised in the range 8-12 wt%.

7. - The composition according to any one of claims 1 to 6, **characterized in that** aluminium chloride hexahydrate is used.

8. - The composition according to any one of claims 1 to 7, **characterized in that** the amount of polyvinyl alcohol is comprised in the range 6-12 wt%.

9. - The composition according to any one of the claims 1 to 8, **characterized in that** the polyvinyl alcohol has a molecular weight comprised between 75 000 and 150 000.

10. - The composition according to any one of claims 1 to 9, **characterized in that** it essentially consists of ferric chloride or a hydrate thereof, aluminium chloride or a hydrate thereof, polyvinyl alcohol, ethanol and water.

11. - The composition according to any one of claims 1 to 9, **characterized in that** it consists of ferric chloride or a hydrate thereof, aluminium chloride or a hydrate thereof, polyvinyl alcohol, ethanol and water.

12. - The composition according to any one of claims 1 to 11, for use in promoting haemostasis, in particular in stopping bleeding and in stimulating wound healing.

13. - The composition for use according to claim 12, **characterized in that** haemostasis is performed after a surgical procedure, for example selected from sutures and biopsies, including skin biopsies, cervical biopsies and vulvar biopsies; or after dental restorative and reconstructive procedures; or after cuts, injuries, wounds or skin erosions.

## Patentansprüche

1. Hämostatische Zusammensetzung, die **dadurch gekennzeichnet ist, dass** sie Folgendes umfasst:
a) Eisenchlorid oder ein Hydrat davon in einer Menge, die im Bereich von 10-25 Gew.-% liegt, als äquivalente Menge Eisenchlorid ausgedrückt;
b) Aluminiumchlorid oder ein Hydrat davon in einer Menge, die im Bereich von 5-20 Gew.-% liegt, als äquivalente Menge Aluminiumchlorid ausgedrückt;
c) Polyvinylalkohol in einer Menge, die im Bereich von 5-15 Gew.-% liegt, und die pharmazeutisch akzeptablen Trägerstoffe Ethanol in einer Menge, die im Bereich von 10-25 Gew.-% liegt, und Wasser,
wobei die Zusammensetzung einen pH-Wert aufweist, der zwischen 1 und 3 liegt,
wobei die Prozentsätze sich auf das Gesamtgewicht der Zusammensetzung beziehen.

2. Zusammensetzung nach Anspruch 1, **dadurch gekennzeichnet, dass** die Menge von Eisenchlorid oder einem Hydrat davon im Bereich von 15-22 Gew.-% liegt.

3. Zusammensetzung nach Anspruch 2, **dadurch gekennzeichnet, dass** die Menge von Eisenchlorid oder einem Hydrat davon im Bereich von 19-21 Gew.-% liegt.

4. Zusammensetzung nach einem der Ansprüche 1 bis 3, **dadurch gekennzeichnet, dass** Eisenchloridhexahydrat verwendet wird.

5. Zusammensetzung nach einem der Ansprüche 1 bis 4, **dadurch gekennzeichnet, dass** die Menge von Aluminiumchlorid oder einem Hydrat davon im Bereich von 7-15 Gew.-% liegt.

6. Zusammensetzung nach Anspruch 5, **dadurch gekennzeichnet, dass** die Menge von Aluminiumchlorid oder einem Hydrat davon im Bereich von 8-12 Gew.-% liegt.

7. Zusammensetzung nach einem der Ansprüche 1 bis 6, **dadurch gekennzeichnet, dass** Aluminiumchloridhexahydrat verwendet wird.

8. Zusammensetzung nach einem der Ansprüche 1 bis 7, **dadurch gekennzeichnet, dass** die Menge von Polyvinylalkohol im Bereich von 6-12 Gew.-% liegt.

9. Zusammensetzung nach einem der Ansprüche 1 bis 8, **dadurch gekennzeichnet, dass** der Polyvinylalkohol ein Molekulargewicht aufweist, das zwischen 75 000 und 150 000 liegt.

10. Zusammensetzung nach einem der Ansprüche 1 bis 9, **dadurch gekennzeichnet, dass** sie im Wesentlichen aus Eisenchlorid oder einem Hydrat davon, Aluminiumchlorid oder einem Hydrat davon, Polyvinylalkohol, Ethanol und Wasser besteht.

11. Zusammensetzung nach einem der Ansprüche 1 bis 9, **dadurch gekennzeichnet, dass** sie aus Eisenchlorid oder einem Hydrat davon, Aluminiumchlorid oder einem Hydrat davon, Polyvinylalkohol, Ethanol und Wasser besteht.

12. Zusammensetzung nach einem der Ansprüche 1 bis 11 zur Verwendung zum Fördern der Hämostase, insbesondere zum Aufhalten von Bluten und Anregen der Wundheilung.

13. Zusammensetzung zur Verwendung nach Anspruch 12, **dadurch gekennzeichnet, dass** Hämostase nach chirurgischem Eingriff, beispielsweise ausgewählt unter Nähten und Biopsien einschließlich Hautbiopsien, Gebärmutterhalsbiopsien und Vulvabiopsien oder nach restaurativen und rekonstruktiven Zahneingriffen oder nach Schnitten, Verletzungen, Wunden und Hauterosionen ausgeführt wird.

## Revendications

1. - Composition hémostatique **caractérisée en ce qu'**elle comprend :
a) du chlorure ferrique ou un hydrate de celui-ci en une quantité comprise dans la plage de 10 à 25 % en poids, exprimée en quantité équivalente de chlorure ferrique ;
b) du chlorure d'aluminium ou un hydrate de celui-ci en une quantité comprise dans la plage de 5 à 20 % en poids, exprimée en quantité équivalente de chlorure d'aluminium ;
c) de l'alcool polyvinylique en une quantité comprise dans la plage de 5 à 15 % en poids et des excipients pharmaceutiquement acceptables éthanol en une quantité comprise dans la plage de 10 à 25 % en poids et de l'eau,
dans laquelle la composition a un pH compris entre 1 et 3
dans laquelle les pourcentages sont rapportés au poids total de la composition.

2. - Composition selon la revendication 1, **caractérisée en ce que** la quantité de chlorure ferrique ou d'un hydrate de celui-ci est comprise dans la plage de 15 à 22 % en poids.

3. - Composition selon la revendication 2, **caractérisée en ce que** la quantité de chlorure ferrique ou d'un hydrate de celui-ci est comprise dans la plage de 19 à 21 % en poids.

4. - Composition selon l'une quelconque des revendications 1 à 3, **caractérisée en ce que** l'on utilise du chlorure ferrique hexahydraté.

5. - Composition selon l'une quelconque des revendications 1 à 4, **caractérisée en ce que** la quantité de chlorure d'aluminium ou d'un hydrate de celui-ci est comprise dans la plage de 7 à 15 % en poids.

6. - Composition selon la revendication 5, **caractérisée en ce que** la quantité de chlorure d'aluminium ou d'un hydrate de celui-ci est comprise dans la plage de 8 à 12 % en poids.

7. - Composition selon l'une quelconque des revendications 1 à 6, **caractérisée en ce que** l'on utilise du chlorure d'aluminium hexahydraté.

8. - Composition selon l'une quelconque des revendications 1 à 7, **caractérisée en ce que** la quantité d'alcool polyvinylique est comprise dans la plage de 6 à 12 % en poids.

9. - Composition selon l'une quelconque des revendications 1 à 8, **caractérisée en ce que** l'alcool polyvinylique a un poids moléculaire compris entre 75 000 et 150 000.

10. - Composition selon l'une quelconque des revendications 1 à 9, **caractérisée en ce qu'**elle est essentiellement constituée de chlorure ferrique ou d'un hydrate de celui-ci, de chlorure d'aluminium ou d'un hydrate de celui-ci, d'alcool polyvinylique, d'éthanol et d'eau.

11. - Composition selon l'une quelconque des revendications 1 à 9, **caractérisée en ce qu'**elle est constituée de chlorure ferrique ou d'un hydrate de celui-ci, de chlorure d'aluminium ou d'un hydrate de celui-ci, d'alcool polyvinylique, d'éthanol et d'eau.

12. - Composition selon l'une quelconque des revendications 1 à 11, destinée à être utilisée pour favoriser l'hémostase, en particulier pour arrêter les saignements et stimuler la cicatrisation des plaies.

13. - Composition destinée à être utilisée selon la revendication 12, **caractérisée en ce que** l'hémostase est réalisée après une intervention chirurgicale, par exemple choisie parmi les sutures et les biopsies, y compris les biopsies cutanées, les biopsies cervicales et les biopsies vulvaires ; ou après des procédures de restauration et de reconstruction dentaires ou après des coupures, des blessures, des plaies ou des érosions cutanées.
